Europäisches Patentamt

European Patent Office

Office européen des brevets

(11) Veröffentlichungsnummer: **0 336 286**
**A2**

(12) ·**EUROPÄISCHE PATENTANMELDUNG**

(21) Anmeldenummer: 89105582.4

(22) Anmeldetag: 30.03.89

(51) Int. Cl.4: **C12N 9/16** , //(C12N9/16,
**C12R1:645)**

(30) Priorität: 02.04.88 DE 3811278

(43) Veröffentlichungstag der Anmeldung:
**11.10.89 Patentblatt 89/41**

(84) Benannte Vertragsstaaten:
**AT BE CH DE ES FR GB GR IT LI LU NL SE**

(71) Anmelder: **BOEHRINGER MANNHEIM GMBH**
**Sandhofer Strasse 116**
**D-6800 Mannheim 31(DE)**

(72) Erfinder: **Bolton, Bryan John**
**7 Chalfont Court Bracken Lane**
**Shirley Southampton(GB)**
Erfinder: **Jarsch, Michael, Dr.rer.nat.**
**Unterkarpfsee 11**
**D-8173 Bad Heilbrunn(DE)**
Erfinder: **Schmitz, Gudrun, Dr.rer.nat.**
**Wettersteinstrasse 3**
**D-8139 Bernried(DE)**
Erfinder: **Kessler, Christoph, Dr.rer.nat.**
**Fraunhoferstrasse 12**
**D-8000 München 5(DE)**

(54) **Typ II-Restriktionsendonuklease Ksp I.**

(57) Die TypII-Restriktionsendonuklease KspI besitzt folgende Erkennungssequenz, spaltet bevorzugt an der durch die Markierung definierten Spaltstelle:

$$5'-CC\ GC|GG-3'$$
$$3'-GG|CG\ CC-5'$$

und ist aus Mikroorganismen der Gattung Kluyvera erhältlich. Sie ist isoschizomer zu SacII und kann zur Erkennung und Spaltung der komplementären doppelsträngigen DNA-Sequenz 5'-CCGCGG eingesetzt werden.

EP 0 336 286 A2

## Typ II-Restriktionsendonuklease KspI

Die Erfindung betrifft die Typ II-Restriktionsendonuklease KspI, ein Verfahren zu ihrer Gewinnung und ihre Verwendung.

Typ II-Restriktionsendonukleasen sind Endodesoxyribonukleasen, welche bestimmte DNA-Sequenzen zu erkennen und zu spalten vermögen. Dabei werden Phosphodiesterbrücken in der Zielsequenz hydrolysiert und zwar in jedem Polynukleotidstrang eine. Typ II-Restriktionsendonukleasen sind daher wertvoll für die Analyse von DNA-Molekülen. Es sind zwar bereits für zahlreiche DNA-Sequenzen spezifische Typ II-Restriktionsendonukleasen bekannt, jedoch ist ihre Zugänglichkeit häufig unbefriedigend. In vielen Fällen sind die Mikroorganismen, aus denen diese Restriktionsendonukleasen isoliert werden, schwer zu kultivieren oder pathogen. Ebenso ist die Ausbeute bei großtechnischer Herstellung oft gering.

Die Restriktionsendonuklease SacII (Gene 47 (1986) 1 -153) ist nur schwierig von SacI zu trennen. Die Ausbeuten sind gering.

Der Erfindung liegt daher die Aufgabe zu Grunde, ein Isoschizomeres zu SacII zur Verfügung zu stellen, welches einfacher und in höherer Ausbeute herstellbar ist.

Gelöst wird diese Aufgabe erfindungsgemäß durch eine Typ-II-Restriktionsendonuklease mit der Erkennungssequenz und der durch die Markierung definierten Spaltstelle

$$5'-CC\ GC|GG-3'\ \cdot$$

$$3'-GG|CG\ CC-5'$$

welche dadurch gekennzeichnet ist, daß sie aus Mikroorganismen der Gattung Kluyvera erhalten wird.

Die erfindungsgemäße neue Restriktionsendonuklease, die im folgenden als KspI bezeichnet wird, hat ein Temperaturoptimum bei ca. 37° C. Das Enzym besitzt eine gute Aktivität zwischen pH 7,2 und pH 7,8 in 10 mmol/l Tris/HCl Puffer mit 10 mmol/l $MgCl_2$ und 1 mmol/l DTE (Dithioerythritol). Das pH-Optimum liegt bei ca. pH 7,5. KspI ist ein Isoschizomer zu SacII. Während jedoch SacII sensitiv gegenüber Methylierung am 1. Cytosin-Rest der Erkennungssequenz CCGCGG ist und somit derart methylierte DNA nicht schneiden kann, ist KspI insensitiv gegenüber Methylierung an dieser Position, d. h. derartig methylierte DNA wird von KspI gespalten.

Die Erkennungssequenz läßt sich durch vollständigen Verdau der DNAs der Viren SV40 und Adeno 2, der Phagen Lambda, phiX1174, den Phagenderivaten M13mp8 und den Plasmiden pBR322 und pBR328 bestätigen. Diese DNA-Moleküle werden mit KspI behandelt.

Tabelle 1 zeigt einen Vergleich der experimentell beobachteten Schnittstellenspezifität mit einer computerermittelten Schnittstellenspezifität für ein Enzym, welches die Sequenz CCGCGG erkennt.

Tabelle I

| DNA | Anzahl der experimentell bestimmten Schnittstellen | Anzahl der durch Computeranalyse bestimmte Schnittstellen | Experimentell bestimmte Fragmentlängen | Durch Computeranalyse bestimmte Fragmentlängen | Spaltpositionen ermittelt durch Computeranalyse |
|---|---|---|---|---|---|
| SV40 | 0 | 0 | 0 | 0 | 0 |
| phiX174 | 1 | 1 | 5400 bp | 5386 bp | bei 2862 bp |
| M13mp8 | 0 | 0 | 0 | 0 | 0 |
| pBR322 | 0 | 0 | 0 | 0 | 0 |
| pBR328 | 0 | 0 | 0 | 0 | 0 |
| bp: Basenpaar(e) | | | | | |

Die Schnittstelle innerhalb der Erkennungssequenz des Enzyms läßt sich wie folgt feststellen: phiX 174-DNA wird mit AatII linearisiert. Das AatII-Fragment wird am 3′-Ende mit terminaler Transferase und [α$^{32}$P]-ddATP $^{32}$P-markiert. Nach einem weiteren Verdau mit AvaII wird ein 2,25 Kb Fragment, welches entweder am AatII-Ende 3′-markiert ist, durch Agarosegel-Elektrophorese isoliert und aus dem Gel gereinigt.

Aliquote dieses Fragmentes werden mit KspI behandelt oder einer basenspezifischen chemischen Spaltung (Maxam and Gilbert, Methods in Enzymology 65, 1980, 499 - 560) zur Sequenzierung unterworfen.

Die Analyse der Fragmente wird durch Sequenzgel Elektrophorese (6 % Polyacrylamid, 8 mol/l Harnstoff) und anschließende Autoradiographie durchgeführt. Die Interpretation der Ergebnisse wird analog Methods in Enzymology 65, 1980, 391 - 401 durchgeführt. Hierbei zeigt sich, daß KspI zwischen Position 2862 und 2863 (Basenpaare) in der Sequenz von phiX 174 mit folgender Spezifität schneidet:

```
5′AAATGCC GC GGATTGG-3′
                |
                |
3′TTTACGG CG CCTAACC-5′
```

Damit ergibt sich als allgemeine Spezifität:

```
5′-CC GC GG-3′
         |
         |
3′-GG CG CC-5′
```

Die experimentell bestimmte Anzahl der Schnittstellen ist identisch mit der Anzahl der Schnittstellen, die durch Computeranalyse mit den verschiedenen DNAs für die Sequenz CCGCGG erhalten wurde (Tabelle I). Zusätzlich wurden diese Daten noch mit den Tabellen aus Gene 10 (1980) 357 - 370, verglichen.

Erfindungsgemäß wird KspI gewonnen, indem man Mikroorganismen der Gattung Kluyvera züchtet und das Enzym aus den Zellen gewinnt. Vorzugsweise wird Kluyvera spec. DSM 4496 verwendet. Zur Gewinnung können die üblichen biochemischen Aufreinigungsmethoden verwendet werden, wobei in den jeweils erhaltenen Fraktionen das Vorhandensein des Enzyms anhand der Spaltung seiner Erkennungssequenz leicht nachgewiesen werden kann. Als Substrat eignet sich beispielsweise Lambda-DNA. Die erhaltenen DNA-Fragmente werden in Agarosegelen in den für die Fragmentauftrennung üblichen Puffersystemen, in Gegenwart von Ethidiumbromid, elektrophoretisch aufgetrennt.

Die für die Gewinnung des Enzyms verwendeten Microorganismen wachsen aerob in Merck Standard I Medium.

Der Mikroorganismuis wurde bei der Deutschen Sammlung von Mikroorganismen, Gesellschaft für biotechnologische Forschung mbH, Mascheroder Weg 16, 3300 Braunschweig, BRD, hinterlegt und trägt die Hinterlegungsnummer DSM 4496. Die optimalen Wachstumsbedingungen sind bei 30°C, pH 7,2 - 7,8. Die Verdoppelungszeit beträgt etwa 2 Stunden.

Das Enzym wird isoliert und gereinigt durch die üblichen chemischen und mechanischen Methoden, beispielsweise durch Hochdruckdispersion, Ultraschall oder enzymatischen Aufschluß. In einer bevorzugten Ausführungsform des erfindungsgemäßen Verfahrens werden die Zellen einem Überdruck von 5 bar ausgesetzt. Die hierbei entstandene Zellenmasse wird in Tris HCl Puffer, pH 8,0, welcher Protease-Inhibitoren enthält, resuspendiert. Anschließend werden die Zellen über eine French-Presse aufgeschlossen und mit Streptomycinsulfat und Ammoniumsulfat präzipitiert. Die weitere Reinigung des Überstands wird vorzugsweise über Affinitätschromatographie, Molekularsiebchromatographie und Ionentauscherchromatographie durchgeführt. Als Material für die Affinitätschromatographie geeignet ist beispielsweise Heparin-Sepharose CL-6B (Pharmacia). Ein geeignetes Molekularsieb ist beispielsweise Ultrogel ACA54 (LKB).

Als Anionentauscher geeignet ist das unter dem Namen DEAE Sepharose Fast Flow (Pharmacia) erhältliche Produkt. Aber auch andere Chromatographiematerialien, die dem Fachmann bekannt sind, sind geeignet.

Die folgenden Beispiele erläutern die Erfindung weiter.

Beispiel 1

Kluyvera spec. DSM 4496 wird bei 30° C 5 Stunden gezüchtet und in der späten logarithmischen bzw. stationären Phase geerntet. Als Kulturmedium wird Merck Standardmedium I verwendet.

Die Zellpaste (30 g Naßgewicht) wird in 1,5 Volumen Puffer A (40 mmol/l Tris-HCl, pH 8,0, 0,1 mmol/l EDTA, 7 mmol/l 2-Mercaptoethanol), welcher Proteaseinhibitoren enthält, resuspendiert. Anschließend werden die Zellen durch zweimalige Passage einer French-Presse bei 23.000 lb/inch² aufgeschlossen. Anschließend wird Streptomycinsulfat zugegeben und der Niederschlag abgetrennt. Der Überstand wird anschließend durch Zugabe von 30 und 60 % (w/v) Ammoniumsulfat behandelt. Der hierbei entstandene Niederschlag wird in 15 ml Puffer B (40 mmol/l Tris-HCl, pH 8,0, 0,1 mmol/l EDTA, 7 mmol/l 2-Mercaptoethanol, 10 % (V-V) Glycerin) aufgelöst. Anschließend wird nach Dialyse gegen Puffer B an eine mit 0,1 mol/l NaCl in Puffer B vorgewaschene Heparin-Sepharose-Säule fraktioniert. Zur Elution wird ein Gradient von 0 - 1 M NaCl verwendet. KspI wird in den Fraktionen zwischen 0,3 und 0,5 M NaCl gefunden. Die aktiven Fraktionen werden mit 80 % Ammoniumsulfat behandelt und der Niederschlag nach Dialyse gegen Puffer B wird an einer Ultrogel AcA54-Säule fraktioniert, welche zuvor mit Puffer B, dem 0,5 mmol/l NaCl zugesetzt wird, equilibriert ist. Die aktiven Fraktionen werden gegen Puffer B dialysiert. Anschließend werden sie auf eine DEAE Sepharose Fast Flow-Säule, welche mit Puffer B equilibriert wurde, aufgegeben. Zur Elution wird ein Gradient von 0 - 0,5 mol/l NaCl in Puffer B verwendet. KspI wird in den Fraktionen zwischen 0,15 und 0,35 M NaCl gefunden.

Die aktiven Fraktionen werden gegen Puffer B dialysiert und auf ein mit 0,2 mol/l NaCl in Puffer B äquilibrierten PC 11-Säule fraktioniert. Zur Elution wird ein Gradient von 0,2 - 1 mol/l NaCl in Puffer B verwendet. KspI wird in den Fraktionen zwischen 0,25 und 0,5 M NaCl gefunden.Die aktiven Fraktionen werden zusammengegeben und gegen Lagerpuffer (20 mmol/l Tris-HCl, pH 8,0, 10 mmol/l 2-Mercaptoethanol und 100 mmol/l NaCl, 50% (v/v) Glycerin) dialysiert.

Beispiel 2

Bestimmung der Aktivität

Definition der Enzymeinheiten:

1 U KspI spaltet 1 μg Lambda-DNA innerhalb 1 Stunde bei 37° C in 25 μl Endvolumen.

Zu einer Mischung von 2,5 μl Inkubationspuffer (80 mmol/l Tris-HCl, pH 7,5/37° C, 100 mmol/l Magnesiumchlorid, 500 mmol/l NaCl und 100 mmol/l 2-Mercaptoethanol) 100 μg/ml RSA (Rinderserumalbumin) werden 1,5 μl Wasser und 5 μl Lambda DNA (optische Dichte: 4 OD/ml) sowie 1 μl KspI-Lösung (1 U/μl) zugegeben. Die Lösung wird eine Stunde bei 37° C inkubiert, auf Eis gekühlt und mit 5 μl eines Abstopp-Reagenses, bestehend aus 7 mmol/l Harnstoff, 20 % (w/v) Saccharose, 60 mmol/l EDTA und 0,01 (w/v) Bromphenolblau versetzt. Anschließend wird eine Trennung durch Elektrophorese in 0,8 % Agarose-Gelen für 3 - 4 Stunden bei 100 V durchgeführt. Die erhaltenen Banden werden über Vergleich mit einem DNA-Längenstandard identifiziert.

## Ansprüche

1. Typ II-Restriktionsendonuklease mit der Erkennungssequenz und der durch die Markierung charakterisierten Schnittstelle

$$5'-CC\ GC\ |\ GG-3'$$
$$3'-GG\ |\ CG\ CC-5'$$

dadurch gekennzeichnet, daß sie insensitiv gegenüber Methylierung am 1. Cytosin-Rest der Erkennungssequenz CCGCGG ist und aus Mikroorganismen der Gattung Kluyvera erhältlich ist.

2. Restriktionsendonuklease nach Anspruch 1 dadurch gekennzeichnet, daß sie aus Kluyvera spec. DSM 4496 erhalten wird.

3. Restriktionsendonuklease nach den Ansprüchen 1 und 2, gekennzeichnet durch ein Temperatur-Optimum bei ca. 37°C und ein pH-Optimum zwischen 7,2 und 7,8.

4. Verfahren zur Gewinnung einer TypII-Restriktionsendonuklease mit der Erkennungssequenz und der durch die Markierung charakterisierten Spaltstelle

$$5'-CC \ GC|GG-3'$$
$$3'-GG|CG \ CC-5'$$

dadurch gekennzeichnet, daß man Mikroorganismen der Gattung Kluyvera züchtet und das Enzym aus den Zellen gewinnt.

5. Verfahren nach Anspruch 4, dadurch gekennzeichnet, daß man Kluyvera spec. DSM 4496 züchtet.

6. Verfahren nach den Ansprüchen 4 und 5, dadurch gekennzeichnet, daß man die Zellen aufschließt und den Zellüberstand gewinnt.

7. Verfahren nach Anspruch 6, dadurch gekennzeichnet, daß man zur Feinreinigung den Zellüberstand einer Affinitätschromatographie, einer Molekularsiebchromatographie, einer Anionenaustauschchromatographie und einer abschließenden Chromatographie über einen Kationenaustauscher unterwirft.

8. Verfahren nach Anspruch 7, dadurch gekennzeichnet, daß man zur Affinitätschromatographie trägerfixiertes Heparin verwendet.

9. Verwendung einer TypII-Restriktionsendonuklease mit der Erkennungssequenz und der durch die Markierung charakterisierten Schnittstelle

$$5'-CC \ GC|GG-3'$$
$$3'-GG|CG \ CC-5',$$

die insensitiv gegenüber methyliertem 1. Cytosin-Rest der Erkennungssequenz CCGCGG ist und aus Mikroorganismen der Gattung Kluyvera erhältlich ist, zur Erkennung und Spaltung der komplementären doppelsträngigen DNA-Sequenz 5'-CCGCGG-3'.